# EUROPEAN PATENT SPECIFICATION

(11) **EP 1 371 982 B1**
(45) Date of publication and mention of the grant of the patent: **24.11.2004**
(21) Application number: 03250869.9
(22) Date of filing: 12.02.2003
(51) Int. Cl.: G01N 33/497, A61B 5/097

(54) **Device and method for measuring alcohol on the breath**
Vorrichtung und Methode zur Messung von Atemalkohol
Dispositif et méthode pour mesurer le taux d'alcool dans l'haleine

(30) Priority: 11.06.2002 DE 10225815
(43) Date of publication of application: 17.12.2003
(73) Proprietor: Dräger Safety AG & Co KGaA, 23560 Lübeck (DE)
(72) Inventor: Stock, Burkhard, 23562 Lubeck (DE); Kruger, Dieter, 23558 Lubeck (DE); Chrzan, Rigobert, 23843 Bad Oldesloe (DE); Busack, Hans-Jurgen, 23560 Lubeck (DE)
(74) Representative: Greenwood, John David

(56) References cited:
- EP-A- 0 510 484
- DE-A- 19 941 586
- US-A- 4 300 384
- US-A- 4 707 336
- US-A- 4 736 619
- US-A- 5 363 857
- US-A- 6 134 462
- US-B1- 6 167 746
- US-B1- 6 289 718

## Description

The invention relates to a device for measuring alcohol on on the breath and a method for measuring alcohol on the breath.

Measurements of alcohol on the breath are known and are carried out with various different measuring devices and methods. A measuring device is disclosed by US6,167,746 B1, for example, which consists of a measuring tube, connected to which, one after another seen in the direction of the gas flow, are a pressure and temperature sensor, as well as a gas sampling valve, with an electrochemical measuring cell connected downstream of this for the measurement of breath alcohol concentrations.

Known breath alcohol measuring devices, such as, for example, the Alcotest® devices, have been used for some years for the specific monitoring of the breath alcohol concentration of motorists, especially at traffic checkpoints.

With the use of breath alcohol measuring devices in combination with what are referred to as "Interlock" systems in private motor vehicles, to prevent driving under the influence of alcohol, the breath alcohol test is not carried out under the supervision of authorised persons.

Accordingly, it is particularly important in this case either to exclude attempts at manipulation during the measurement of the breath alcohol, and/or to detect attempts of this nature by the registration of characteristic measured values, for example with the read-out of operational data at an authorised service station. The measurement result for the breath alcohol concentration can, for example, be manipulated by the air expelled by the breath being conducted via a wash bottle or through filter materials, by air from an air reservoir being introduced into the measuring device, or the like.

US-A-5,363,857, US-B1-6,167,746 and US-A-4,736,619 are prior art document to this application.

US-A-5,363,857 discloses a device and method for measuring the CO₂ concentration including a flow chamber which is provided with a flow diaphragm, a differential pressure sensor having first and second measurement connections, a CO₂ sensor, a choke element and an evaluation and control unit. However, pressure measurements and gas concentration measurements are performed in two separate flow circuits.

US-B1-6,167,746 discloses a device and method for measuring the alcohol concentration in a gaseous mixture including a flow chamber, a pressure sensor and a fuel cell for the measurement of the alcohol gas concentration. No differential pressure measurements are performed due to the lack of a diaphragm in the flow chamber.

The object of the invention is to provide an improved device and a method for the measurement of alcohol on the breath, so that functional faults can be identified and more precise measurement results obtained.

This object is achieved with the features of claim 1 for the device according to the invention and the features according to claim 6 for the method according to the invention.

The dependent claims represent optional features of the device according to claim 1 or the method according to claim 6.

A substantial advantage of the ammgement according to the invention lies in the possibility of the detection of various different function faults and operational states with the aid of a single device with the circuit arrangement of the components indicated.

An additional advantage is provided if devices according to the invention are used in motor vehicles, aircraft, or watercraft in order to block operation as a function of the concentration of the alcohol measured on the breath of the operator. As an alternative the starter unit of a drive engine or motor is only released for operation if a comparison of the measured value or values for the concentration of alcohol on the breath indicates that said values do not exceed predetermined reference values.

In the broadest sense, the present invention can be used in such a way that a machine, ie. a motor or a closure device, is either cleared for operation by a device for measuring the alcohol on the breath located upstream in the circuit as a function of the result of a measurement of the alcohol on the breath of an operating person and after comparison with predetermine reference values, namely if the reference values are not exceeded, or blocked for further operation, namely if the predetermined reference values are exceeded after measurement at a specific moment of time or at different moments of time after the initial clearance for operation of the machine.

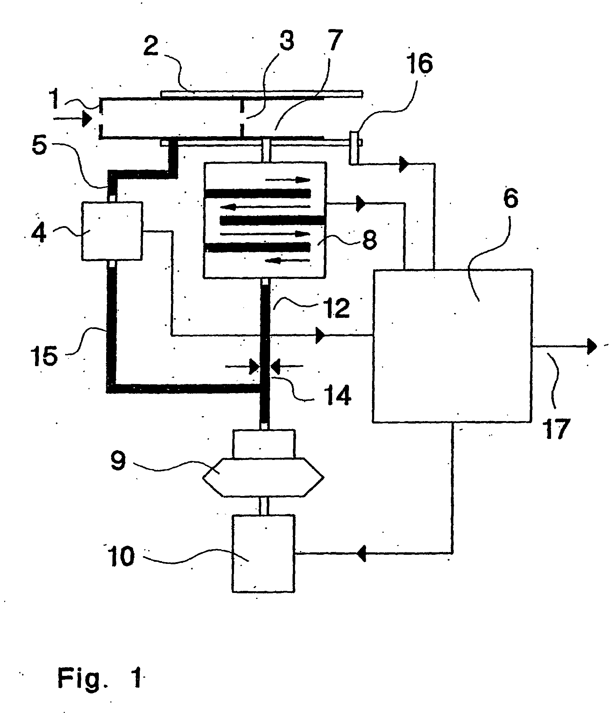

An exemplary embodiment of the invention is represented hereinafter with the aid of the single figure, Figure 1, which represents a device according to the invention in diagrammatic form which will now be described.

A replaceable mouthpiece 1 is inserted into a tubular flow chamber 2, into which the person to be tested blows, on whose breath the concentration of alcohol is to be measured. At a flow diaphragm 3, with a circular flow cross-section of some 3 to 4 millimetres, the flow (breath gas volume flow) creates a pressure drop, hitherto dependent on legal provisions. The pressure drop is measured with a differential pressure sensor 4, of which the first measurement connection is connected via a first gas line 5 to the mouthpiece 1 via the interior space of the flow chamber 2 upstream of the flow diaphragm 3.

The second measurement connection of the differential pressure sensor 4 is effected via the breath alcohol sensor 8, and therefore has a connection to the interior space of the flow chamber 2 downstream, ie. behind the flow diaphragm 3. An evaluation and control unit 6 calculates from the measured differential pressure signal the breath gas flow volume which is flowing through the mouthpiece 1 and the flow chamber 2, and from this calculates in turn the volume of breath gas emitted by the person be measured by integration over the period of time concerned. Behind the flow diaphragm 3, ie. downstream of it, is located the intake nozzle 7 of a breath alcohol sensor 8, through which, with the aid of a specimen acquisition system 9, 10, consisting of the elastic concertina element 9 and the pressure magnet 10, a specimen is drawn from the breath gas flow into the breath alcohol sensor 8, as soon as the person whose breath alcohol concentration is to be measured has emitted a certain minimum breath gas volume.

At the start of the taking of the specimen, a flow pulse is issued by the evaluation and control unit to the pressure magnet 10, which, as a result, compresses the concertina element 9. When the current is switched off, the concertina element 9 relaxes once again, and in the process sucks in a volume of air, defined by the design of the system, through the breath alcohol sensor 8, where the alcohol is rapidly absorbed by the sensor surface of an electrochemical gas sensor which is used for preference, and, by means of a characteristic electrochemical attestation reaction leads to a concentration-dependent measurement signal for the breath alcohol content of the person being measured and which can be further evaluated in a known manner.

The breath alcohol sensor 8 is connected via a second gas line 12 to the specimen acquisition system 9, 10. Located in this second gas line 12 is a choke element 14 with a cross-sectional diameter of just one millimetre, at which a pressure change pertains when the concertina element 9 is compressed or relaxes. Such pressure changes pass via the third gas line 15 to the second measurement connection of the differential pressure sensor 4 and can therefore be measured. A temperature sensor 16 arranged downstream of the flow diaphragm 3 at the outlet of the measurement chamber 2 serves to check whether breath gas is in fact flowing through the mouthpiece 1 and the measuring chamber 2. To achieve this, a measurement is made of whether the temperature at the temperature sensor 16 is changing when the differential pressure sensor 4 indicates that a flow pertains.

In a preferred embodiment of the breath alcohol measuring device, the evaluation and control unit 6 is connected on the output side via a signal connection 17 to a machine or device to be operated by a person, the person, the operation of which is either cleared or blocked as a function of the breath alcohol concentration of the operating person measured previously in the breath alcohol measuring device and after comparison with corresponding reference values by the evaluation and sensor unit 6. In particular, the starter of a drive engine or motor of a motor vehicle, aircraft, or watercraft will only be cleared for operation via the signal line 17 if the breath alcohol value measured falls short of a predetermined limit value, or at least does not exceed it. As an alternative, the machine which is to be operated, in particular the starter or the drive engine or motor, will be blocked for further operation if predetermined reference values for the breath alcohol concentration, after measurement at a specific moment of time or at different moments of time, is exceeded after an initial clearance for operation of the machine.

Before the actual acquisition of the sample and measurement of the breath alcohol content, the breath alcohol measuring device checks whether the specimen acquisition system 9, 10 is functioning correctly. To do this, the measurement signal of the different pressure sensor 4 is evaluated by the evaluation an control unit 6. By switching on the current for the pressure magnet 10, the concertina element 9 is compressed. After about 100 milliseconds, the current is switched off and the concertina element 9 relaxes. Due to the rapid compression the measured differential pressure over time initially rises sharply, runs through a characteristic maximum, and then again reverts to zero when the pressure magnet 10 has reached its end stop. If the concertina element 9 then relaxes, an under-pressure pertains at the flow diaphragm 3, which has a similar temporal characteristic curve to the compression pulse, but with negative pressure imposition. The relaxation process is concluded when the concertina element 9 is completely relaxed. The maximum amount of the compression pulse acquired via the measured differential pressure is perceptibly greater than the relaxation pulse, likewise detected by means of the measured differential pressure, since the force compressing the concertina element 9 is greater than its reversion force.

For a function test of the specimen acquisition system 9, 10, a check is made by a comparison with stored reference signals by the evaluation and control unit 6 as to whether both the compression and the relaxation signal are present, for example, if the compression pulse is missing after the current has been switched on, or if this is perceptibly smaller in relation to the reference signal, ie. less than 50%, for example, the concertina element 9 has not moved or has a leak. If there is indeed a compression pulse present, but not the relaxation pulse, the concertina element 9 is blocked in its stop position. In both cases, the evaluation and control unit 6 will issue a fault signal.

Another possible fault to be detected arises if the intake nozzle 7 of the breath alcohol sensor 8 is blocked due to manipulation or dirt contamination. In this case, when the specimen acquisition system 9, 10, is actuated, no specimen will be drawn out of breath flow into the breath alcohol sensor 8, so that the time-dependent differential pressure characteristics of the concertina element 9, measured by the differential pressure sensor 4, will differ perceptibly from the normal. Because the compressed gas volume cannot escape, a higher maximum pressure builds up, and will not drop again unless the current of the pressure magnet 10 is interrupted after about 100 milliseconds and the concertina element 9 relaxes. The precise pressure characteristic depends on how leak-tight the intake nozzle 7 really is. An impermissible sample taking is defined, for example, in that the half-value width of the compression pulse should not exceed a stored reference value. Another known manipulation possibility of the alcohol measuring device is the closure of the outlet aperture of the flow chamber 2, in order for only a static pressure to build up without a flow pertaining. However, because the pressure then takes effect on both measurement connections of the differential pressure sensor 4, no measurement signal pertains, and therefore no flow is identified either, so that no sample taking or measurement occurs. This is a further substantial advantage of the arrangement according to the invention.

Another known manipulation possibility, namely the closure of the flow aperture 3 in the flow chamber 2, can be identified with the aid of a temperature sensor 16, used for preference. With this manipulation possibility, a pressure pertains in the front part of the flow chamber 2, without the breath air passing at the intake nozzle 7 of the breath alcohol sensor 8. The differential pressure sensor 4 in this case detects a differential pressure, so that, as with a normal measurement, a flow is detected. If, after a minimum volume has been attained, the taking of a sample is started by the evaluation and control unit 6, only external air passes into the breath alcohol sensor 8. Accordingly, the temperature sensor 16 serves to determine whether breath air is flowing into the rear downstream part of the flow chamber 2. To achieve this, the temperature in the flow chamber 2 is measured immediately before the issue of the breath gas sample by the person who is to be measured, and thereafter the time-dependent temperature characteristic is measured during the exhalation process. Experience has shown that the breath gas temperature during the exhalation process is never constant, so that the detection of a slight temperature change of less than one degree Celsius already indicates that exhalation gas has flowed past at the temperature sensor 16, and therefore also at the intake nozzle 7 of the breath alcohol sensor 8. Conversely, no temperature change is measured with a closed flow diaphragm 3.

## Claims

1. Device for measuring breath alcohol concentration on the breath including:
a flow chamber (2) for taking in the exhaled breath gas volume flow from a person who is to be measured and which is provided with a flow diaphragm (3);
a differential pressure sensor (4) having a first measurement connection which is connected via a first gas line (5) to the interior of the flow chamber (2) upstream of the flow diaphragm (3), and having a second measurement connection which is connected via second gas line (12), a third gas line (15) and a breath alcohol sensor (8) to the interior of the flow chamber (2) downstream of the flow diaphragm (3);
a choke element (14) located in the second gas line (12) the second gas line (12) being connected to a sample acquisition system (9, 10);
an evaluation and control unit (6) having inputs connected to the sensors (4, 8) and which receive the measurement signals from them, the evaluation and control unit (6) having an output connected to the sample acquisition system (9, 10) for the purpose of controlling it.

2. Device according to claim 1, in which the flow chamber (2) in the outlet area downstream of the flow diaphragm (3) has a temperature sensor (16), which is connected to the evaluation and control unit (6) for the transfer of the temperature measured values.

3. Device according to claim 1 or 2, in which the sample acquisition system (9, 10) consists of an electrically-drive pump element, in particular of a concertina element (9) actuated by a pressure magnet (10).

4. Device according to at least one of the foregoing claims, in which evaluation and control unit (6) is connected on the output side by means of a signal connection (17) to a device which releases or blocks the operation of a machine or device, in particular to the starter unit of the drive engine or motor of a motor vehicle, aircraft, or watercraft.

5. Device according to at least one of the foregoing claims, in which the breath alcohol sensor (8) is an electrochemical, semiconductor, or infrared-optical gas sensor.

6. Method for the measurement of breath alcohol concentration on the breath using a device according to any one of the foregoing claims, in which:
a) the differential pressure arising across the flow diaphragm (3) after the issue of a breath gas sample by the person who is to be measured, is measured;
b) values for the breath gas volume flow allocated to the differential pressure measured are determined in the evaluation and control unit (6), and are compared with stored reference values, so that
c) an activation of the sample acquisition system (9, 10) only takes place if a differential pressure which differs from zero is present, and
d) the integration over a predetermined period of time of the values for the breath gas volume flow, allocated to the measured differential pressure, attains a predetermined stored minimum breath gas volume.

7. Method according to claim 6, in which, before the start of a measurement, in a function test for the sample acquisition system (9, 10) the signal characteristic curve of the differential pressure, acquired by the differential pressure sensor (4), is compared over the period of time before and after actuation of the sample acquisition system (9, 10) with stored reference signals, and in the event of their exceeding or falling short of these, a signal is issued via the evaluation and control unit (6).

8. Method according to claim 6 or 7, in which the temperature of the breath gas is detected by means of the temperature sensor (16) and deviations from the specified referenced values lead to a blocking of the sample acquisition system (9, 10) and/or to the issue of a signal by the evaluation and control unit (6).

## Patentansprüche

1. Gerät zur Messung des Atemalkoholgehalts im Atem einschließlich:
einer Durchflusskammer (2) zur Aufnahme des ausgeatmeten Atemgasvolumenstroms einer Person, die beurteilt werden soll, und die mit einer Durchflussblende (3) ausgestattet ist,
einem Differenzdruckmesssensor (4) mit einer ersten Messleitung, die über eine erste Gasleitung (5) mit dem Innern der Durchilusskammer (2) Stromauf der Durchflussblende (3), verbunden ist, und mit einer zweiten Messleitung, die über eine zweite Gasleitung (12), eine dritte Gasleitung (15) und einen Atemalkoholmesssensor (8) mit dem Inneren der Durchflusskammer (2), Stromab der Durchflussblende (3), verbunden ist,
einem Drosselelement (14), das in der zweiten Gasleitung (12) angeordnet ist, wobei die zweite Gasleitung (12) an ein Probenerfassungssystem (9, 10) angeschlossen ist,
eine Auswerte- und Steuereinheit (6) mit Eingängen, angeschlossen an die Messsensoren (4, 8), die das Messsignal von diesen aufnehmen, wobei die Auswerte- und Steuereinheit (6) eine Ausgang ausweist, der an das Probenerfassungssystem (9, 10) angeschlossen ist, um dieses zu regeln.

2. Gerät nach Anspruch 1, **dadurch gekennzeichnet, dass** die Durchflusskammer (2) am Auslassbereich Stromab der Durchflussblende (3) einen Temperatursensor (16) aufweist, der an die Auswerte- und Steuereinheit (6) angeschlossen ist, um die gemessenen Temperaturwerte zu übertragen.

3. Gerät nach Anspruch 1 oder 2, **dadurch gekennzeichnet, dass** das Probenerfassungssystem (9, 10) aus einem elektrisch angetriebenen Pumpelement, speziell aus einem Übergangsbalgelement (9), angetrieben durch einen Druckmagneten (10), besteht.

4. Gerät nach zumindest einem der vorangegangenen Ansprüche, **dadurch gekennzeichnet, dass** die Steuereinheit (6) an der Ausgangsstelle durch eine Signalverbindung (17) an eine Baugruppe angeschlossen ist, welche die Funktion einer Maschine oder eines Gerätes blockiert oder freigibt, speziell an die Startereinheit eines Antriebsmotors eines Kraftfahrzeugs, Flugzeugs oder Wasserfahrzeugs.

5. Gerät nach zumindest einem der vorangegangenen Ansprüche, **dadurch gekennzeichnet, dass** der Atemalkoholmesssensor (8) ein elektrochemischer Sensor, Halbleitersensor oder Infrarot Gassensor ist.

6. Verfahren zur Messung der Atemalkoholkonzentration im Atem unter Verwendung eines Geräts gemäß einem der vorangegangenen Ansprüche, **dadurch gekennzeichnet, dass** man:
a) den Differenzdruck, der sich an der Durchflussblende (3) einstellt, nach dem die Person, die beurteilt werden soll, das Atemgas emittiert hat, misst,
b) dem Atemgasvolumenstrom anhand der gemessenen Druckdifferenz Werte zugeordnet, die in der Auswerte- und Steuereinheit (6) bestimmt und mit gespeicherten Vergleichswerten verglichen werden, so dass
c) eine Aktivierung des Probenerfassungssystems (9,10) nur stattfindet, wenn der Druckunterschied von Null verschieden ist und
d) die Integration der Werte für den Atemgasvolumenstrom, zugeordnet durch den gemessenen Druckunterschied über eine zuvor bestimmte Zeitdauer, ein zuvor bestimmtes, gespeichertes Minimumatemgasvolumen erreicht.

7. Verfahren nach Anspruch 6, **dadurch gekennzeichnet, dass** vor dem Start der Messung bei einem Funktionstest des Probenerfassungssystems (9, 10) die Signalkennlinie des Differenzdrucks, erhalten vom Differenzdruckmesssensor (4), während der Zeitdauer vor und nach der in Betriebnahme des Probenerfassungssystems (9,10), mit gespeicherten Referenzsignalen verglichen wird, und für den Fall, dass diese diese über- oder unterschreiten, über die Auswerte- und Steuereinheit (6) ein Signal abgegeben wird.

8. Verfahren nach Anspruch 6 oder 7, **dadurch gekennzeichnet, dass** die Temperatur des Atemgases mit Hilfe eines Temperatursensors (16) bestimmt wird, und Abweichungen von den Referenzwerten zu einem blockieren des Probennahmesystems (9, 10) und / oder zur Abgabe eines Signals durch die Auswerte- und Steuereinheit führen.

## Revendications

1. Appareil pour mesurer la concentration d'alcool dans le souffle, comprenant:
une chambre d'écoulement (2) pour prélever le flux volumique gazeux du souffle expiré d'une personne que l'on veut mesurer, ladite chambre étant pourvue d'un diaphragme d'écoulement (3);
un capteur de pression différentielle (4) ayant une première connexion de mesure qui est connecté, via un premier conduit de gaz (5), à l'intérieur de la chambre d'écoulement (2) en amont du diaphragme d'écoulement (3) et ayant une deuxième connexion de mesure qui est connectée via un deuxième conduit de gaz (12), un troisième conduit de gaz (15) et un capteur d'alcool du souffle (8) à l'intérieur de la chambre d'écoulement (2) en aval du diaphragme d'écoulement (3);
un élément d'étranglement (14) situé dans le deuxième conduit de gaz (12), le deuxième conduit de gaz (12) étant connecté à un système d'acquisition d'échantillons (9, 10);
une unité d'évaluation et de commande (6) possédant des entrées connectées aux capteurs (4, 8) et qui en reçoivent les signaux de mesure, l'unité d'évaluation et, de commande ayant une sortie raccordée au système d'acquisition d'échantillons (9, 10) dans le but de le commander.

2. Appareil selon la revendication 1, dans lequel une chambre d'écoulement (2) dans la zone de sortie en aval du diaphragme d'écoulement' (3) a un capteur de température (16) qui est connecté à l'unité d'évaluation et de commande (6) pour le transfert des valeurs de température mesurées.

3. Appareil selon la revendication 1 ou 2, dans lequel le système d'acquisition d'échantillons (9, 10) est constitué d'un élément de pompe à commande électrique, en particulier d'un élément en accordéon (9) actionné par un aimant de pression (10).

4. Appareil selon au moins l'une des revendications précédentes, dans lequel l'unité d'évaluation et de commande (6) est connectée sur le côté de sortie au moyen d'une connexion de signal (17) à un appareil qui libère ou bloque le fonctionnement d'une machine ou d'un appareil, en particulier à l'unité de démarrage du moteur d'entraînement d'un véhicule à moteur, d'un aéronef ou d'un navire.

5. Appareil selon au moins l'une des revendications précédentes, dans lequel le capteur d'alcool du souffle (8) est un capteur de gaz électrochimique, semiconducteur ou optique infrarouge.

6. Procédé pour mesurer la concentration d'alcool du souffle en utilisant un appareil selon l'une quelconque des revendications précédentes, dans lequel :
a)la pression différentielle se trouvant en travers du diaphragme d'écoulement (3), après l'émission d'un échantillon de gaz du souffle par la personne qui doit être mesurée, est mesurée ;
b) les valeurs pour l'écoulement volumique gazeux du souffle allouées à la pression différentielle mesurée dans l'unité 'd'évaluation et de commande (6) sont déterminées et comparées à des valeurs de référence stockées,
c) de sorte qu'une activation du système d'acquisition d'échantillons (9, 10) a lieu si une pression différentielle différant de zéro est présente, et
d) que l'intégration sur une période de temps prédéterminée des valeurs pour l'écoulement volumique gazeux du souffle, allouées à la pression différentielle mesurée, atteigne un volume gazeux du souffle minimal stocké prédéterminé.

7. Procédé selon la revendication 6, dans lequel, avant le démarrage d'une mesure, dans un test de fonctionnements pour le système d'acquisition d'échantillon (9, 10), la courbe caractéristique du signal de la pression différentielle, acquise par le capteur de pression différentielle (4), est comparée sur la période de temps avant et après l'actionnement du système d'acquisition d'échantillon (9, 10) à des signaux de référence stockés et, dans le cas de leur dépassement ou de leur insuffisance, un signal est délivré via l'unité d'évaluation et de commande (6).

8. Procédé selon la revendication 6 ou 7, dans lequel la température du gaz du souffle est détectée au moyen du capteur de température (16) et des écarts par rapport aux valeurs de référence spécifiées mènent à un blocage du système de prélèvement d'échantillon (9, 10) et/ou à la délivrance d'un signal par l'unité d'évaluation et de commande (6).
